# EUROPÄISCHE PATENTSCHRIFT

(11) **EP 0 754 174 B1**
(45) Veröffentlichungstag und Bekanntmachung des Hinweises auf die Patenterteilung: **23.12.1998**
(21) Anmeldenummer: 95914317.3
(22) Anmeldetag: 28.03.1995
(51) Int. Cl.: C07C 303/22, C07C 309/58

(54) **VERFAHAREN ZUR HERSTELLUNG VON CARBOXY-ARENSULFONSÄUREN UND DEREN CARBONSÄUREDERIVATEN**
PROCESS FOR PRODUCING CARBOXY ARENE SULPHONIC ACIDS AND THEIR CARBOXYLIC ACID DERIVATIVES
PROCEDE DE FABRICATION D'ACIDES CARBOXYARENESULFONIQUES ET LEURS DERIVES DU TYPE ACIDE CARBOXYLIQUE

(30) Priorität: 05.04.1994 DE 4411682
(43) Veröffentlichungstag der Anmeldung: 22.01.1997
(73) Patentinhaber: Hoechst Schering AgrEvo GmbH, 13509 Berlin (DE)
(72) Erfinder: VERMEHREN, Jan, D-65510 Idstein (DE)
(86) Internationale Anmeldenummer: EP9501155
(87) Internationale Veröffentlichungsnummer: WO9526952

(56) Entgegenhaltungen:
- US-A- 4 145 349
- JOURNAL OF ORGANIC CHEMISTRY, Bd. 45, Nr. 12, 6.Juni 1980 WASHINGTON, DC, US, Seiten 2365-2368, K. NAGIRA, ET AL.: 'Reaction of diazonium salts with transition metals. 4. Palladium(0)-catalysed carboxylation of arenediazonium salts' in der Anmeldung erwähnt

## Beschreibung

Verbindungen der allgemeinen Formel (A) oder deren Salze

Y-SO₂-Ar-Z (A)

worin
- Ar: einen unsubstituierten oder substituierten Arylenrest,
- Y: OH, Amino, substituiertes oder disubstituiertes Amino oder Halogen und
- Z: eine Carboxygruppe oder die funktionelle Gruppe eines Carbonsäurederivats
bedeuten,
können als Zwischenprodukte bei der Herstellung von Arzneimitteln, Pflanzenschutzmitteln und Farbstoffen eingesetzt werden (siehe z.B. EP-A- 007687, (US-A-4,383,113) WO 92/13845 und Handbücher zur chemische Synthese).

Die Herstellung der Verbindungen der Formel (A) ist aufgrund der Zahl reaktiver funktioneller Gruppen bislang nur mit relativ vielstufigen Synthesesequenzen möglich. Prinzipiell unterscheiden sich Verfahren zur Herstellung von Verbindungen der Formel (A) in Reihenfolge und Methode der Einführung von funktionellen Gruppen am aromatischen Kernbaustein.

So ist aus WO 92/13845 beispielsweise ein Herstellungsverfahren von Carbalkoxy-iodbenzolsulfochloriden bekannt, in dem die SO₂Cl-Gruppe in der letzten Synthesestufe durch Umsetzung der Diazoniumsalze von Aminoiodbenzoesäurealkylestern mit SO₂ in Gegenwart von Kupferchloriden eingeführt wird. Im Falle der ortho-Stellung der Aminogruppe zur Carbalkoxygruppe tritt außerdem als Zwischenprodukt zunächst ein Disulfid auf, das zur Überführung in das Sulfochlorid zusätzlich mit Chlor umgesetzt werden muß. Die eingesetzten Amino-iodbenzoesäurealkylester sind zwar nach Standardverfahren auch in größeren Mengen zugänglich, erfordern jedoch vielstufige Synthesewege, wenn sie von wohlfeilen Verbindungen aus hergestellt werden sollen.

Wenn die Carbonsäurefunktion am Ende der Synthesekette eingeführt werden soll, bieten sich prinzipiell Carbonylierungsmethoden an. Beispielsweise sind metallkatalysierte, insbesondere palladiumkatalysierte Carbonylierungen von Arylhalogeniden und von Aryldiazonium-tetrafluorboraten bekannt, wobei Ester von aromatischen Carbonsäuren bzw. nach deren Hydrolyse die freien Carbonsäuren erhalten werden (siehe z. B.: J. Org. Chem. **39**, 3318 (1974); J. Org. Chem. **45**, 2365 (1980); JP-A-01-316364; G. A. Olah et al., Synlett **1990**, 596). Die bekannten Methoden zur katalytischen Carbonylierung weisen aber eine Reihe von Nachteilen auf:
- Häufig mißlingen die Carbonylierungen von Arylhalogeniden besonders bei Verbindungen, welche weitere funktionelle Gruppen, wie z.B. lod, enthalten, weil keine ausreichende Selektivität erhalten werden kann.
- Die in der genannten alternativen Methode für Carbonylierungen eingesetzten Aryldiazonium-tetrafluorborate sind aufgrund der unter anderem aus Stabilitätsgründen erforderlichen BF₄-Gruppe verhältnismäßig teuer bzw. schlecht zugänglich, was deren technischen Einsatz einschränkt. Die Anwendbarkeit der Methode wird außerdem durch manche funktionelle Gruppe im Molekül oder Lösungsmittel beeinträchtigt; beispielsweise führt die Anwesenheit eines Alkohols zu unbefriedigenden Ausbeuten und Nebenprodukten (J. Org. Chem. **45**, 2365 (1980)).

Es bestand somit die Aufgabe, ein industriell anwendbares Verfahren zur Herstellung von Verbindungen der Formel (I) bereitzustellen, dessen Ausgangsmaterialien gut zugänglich sind und das die obengenannten Nachteile teilweise oder ganz vermeidet.

Überraschend wurde nun ein verbessertes Verfahren vom Typ der katalytischen Carbonylierung von Diazoniumsalzen von aromatischen Aminoverbindungen gefunden, das die genannte Aufgabe löst.

Gegenstand der Erfindung ist ein Verfahren zur Herstellung von Verbindungen der allgemeinen Formel (I) oder deren Salzen,

HO₃S-Ar-CO-Nuc (I)

worin
- Ar: einen unsubstituierten oder substituierten Arylenrest, vorzugsweise einen unsubstituierten oder substituierten 1,2-, 1,3- oder 1,4-Phenylenrest, und
- Nuc: den Rest eines Nukleophils Nuc-H
bedeuten, dadurch gekennzeichnet, daß man ein inneres Diazoniumsalz der Formel (II)

⁻O₃S-Ar-N₂⁺ (II)

mit Kohlenmonoxid in Gegenwart eines Metallkatalysators aus der Gruppe, die aus Katalysatoren der Metalle der Gruppe VIII des Periodensystems und des Kupfers besteht, und dem Nucleophil der Formel Nuc-H oder dessen Salz umsetzt.

In den obigen Formeln (I) und (II) und im folgenden bedeutet Ar einen gegebenenfalls substituierten Arylenrest, wobei der aromatische Teil ein carbocyclischer aromatischer, vorzugsweise sechsgliedriger Ring ist, der gegebenenfalls benzokondensiert ist. Beispiele für Ar sind gegebenenfalls substituiertes Phenylen und Naphthylen. Als Substituenten am aromatischen Kern kommen ein oder mehrere Reste in Frage, die allgemein an aromatischen Resten möglich und vorzugsweise bekannt oder üblich sind. Beispiele für solche Substituenten sind Alkyl, Alkenyl, Alkinyl, Alkoxy, wobei die letztgenannten vier Reste unsubstituiert oder duroh ein oder mehrere Halogenatome oder einen oder mehrere Alkoxyreste substituiert sind, und unsubstituiertes oder substituiertes Aryl, unsubstituiertes oder substituiertes Arylalkyl, Halogen, Nitro, Cyano, Sulfo, Acyl, Alkylsulfonyl, Haloalkylsulfonyl, Alkylthio, unsubstituiertes oder substituiertes Aryloxy, Hydroxy, Monoalkylamino, Dialkylamino und Acylamino. Bei den C-haltigen Substituenten sind solche mit 1 bis 6 C-Atomen, vorzugsweise 1 bis 4 C-Atomen bevorzugt. Ausgenommen sind naturgemäß Häufungen oder Kombinationen von Substituenten, die mit der Stabilität der Verbindungen (I) und (II) unvereinbar sind.

Aryl auch in zusammengesetzten Bedeutungen wie Arylalkyl oder Aryloxy, bedeutet einen aromatischen Rest, beispielsweise Phenyl oder Naphthyl. Die genannten Substituenten am aromatischen Kern von Ar kommen prinzipiell auch als Substituenten für Aryl oder am Arylteil in Arylalkyl oder Aryloxy in Frage, wobei unüberschaubare Molekülgrößen für (I) und (II) vermieden werden sollten, d. h. Arylalkyl, das durch Arylalkyl substituiert ist, oder durch Phenoxy substituiertes Phenoxy kann lediglich in Einzelfällen sinnvoll sein.

Acyl bedeutet beispielsweise Formyl, Alkylcarbonyl, Alkoxycarbonyl, unsubstituiertes oder substituiertes Carbonamid, wie N-Monoalkylcarbonamid und N,N-Dialkylcarbonamid, wobei in den zusammengesetzten Bedeutungen Alkyl mit 1 bis 6 C-Atomen, insbesondere 1 bis 4 C-Atomen, bevorzugt ist; weiterhin bedeutet Acyl beispielsweise Arylcarbonyl, wie z. B. unsubstituiertes oder substituiertes Benzoyl. Besonders bevorzugt bedeutet Acyl (C₁-C₄-Alkyl)carbonyl.

Der Begriff "inneres Diazoniumsalz" der Formel (II) schließt neben den Verbindungen ohne Fremdkationen und -anionen auch solche ein, in denen die Sulfonatgruppe mit Fremdkationen, wie z. B. Na⁺ und K⁺, und die Diazoniumgruppe mit Fremdanionen, wie z. B. Cl⁻ und ein Äquivalent SO₄⁻⁻, koordiniert sind.

Bevorzugt bedeutet Ar einen 1,2-, 1,3- oder 1,4-Phenylenrest, der unsubstituiert oder durch einen oder zwei Reste aus der Gruppe C₁-C₄-Alkyl, C₁-C₄-Haloalkyl, C₂-C₄-Alkenyl, C₂-C₄-Alkinyl, C₁-C₄-Alkoxy, Benzyl, Halogen, Nitro, Cyano, (C₁-C₄-Alkyl)-carbonyl, C₁-C₄-Alkylsulfonyl, C₁-C₄-Alkylthio, Phenoxy, Hydroxy, Mono-(C₁-C₄-alkyl)-amino und Di-(C₁-C₄-alkyl)-amino substituiert ist. Von besonderem Interesse ist 1,2-, 1,3- oder 1,4-Phenylen, das unsubstituiert oder durch einen oder zwei Reste aus der Gruppe Methyl, Ethyl, Methoxy, Ethoxy, Halogen, wie Fluor, Chlor, Brom und lod, und Nitro, Cyano, (C₁-C₃-Alkyl)carbonyl, wie Acetyl, und C₁-C₄-Alkylsulfonyl, wie Methyl- oder Ethylsulfonyl, und Hydroxy und Di-(C₁-C₄-alkyl)-amino, wie Dimethylamino, substituiert ist; Halogen ist dabei besonders bevorzugt.

Nuc bedeutet den Rest des Nucleophils Nuc-H oder dessen Salz, das erfindungsgemäß mit dem inneren Diazoniumsalz (II) umgesetzt wird. Als Nucleophil kommt eine breite Palette strukturell verschiedenster Verbindungen in Frage, die jedoch ein austauschfähiges Wasserstoffatom ("acides H") oder, im Falle des Salzes, ein austauschfähiges Kation aufweisen müssen. Als übliche Nucleophile sind beispielsweise Verbindungen mit reaktionsfähigen funktionellen OH-, NH- oder SH-Gruppen oder dessen Salze bekannt und in der Regel erfindungsgemäß einsetzbar.

Die Nucleophile Nuc-H bzw. deren Salze sind dann z. B. Verbindungen aus der Gruppe enthaltend Alkohole und deren Salze, primäre und sekundäre Amine, Mercaptane, Carbonsäuren und deren Salze; aber auch Ammoniak kommt als Nucleophil in Betracht. Wasser ist als Nucleophil Nuc-H meist weniger geeignet, weil Wasser in der Regel zu Nebenreaktionen mit dem Diazoniumsalz (II) führt. In Einzelfällen, unter Verwendung geeigneter Lösungsmittelkombinationen, kommt auch Wasser als Nucleophil Nuc-H in Frage. Beispiele für reaktionsfähige Verbindungen Nuc-H sind
- aliphatische Alkohole, wie die Alkanole Methanol, Ethanol, n- und i-Propanol, n-, i-, t- und 2-Butanol, und aromatische Alkohole, wie unsubstituiertes und substituiertes Phenol oder Naphthol, sowie deren Salze,
- aliphatische Amine, wie Methylamin, Dimethylamin, Morpholin, und aromatische Amine, wie unsubstituiertes und substituiertes Anilin,
- Alkylthioverbindungen, wie Methylmercaptan, und
- aliphatische und aromatische Carbonsäuren und vorzugsweise deren Salze, wie Ameisensäure und Formiate, Essigsäure und Acetate, Propionsäure und Propionate, Benzoesäure und Benzoate.

### Beispielsweise bedeutet dementsprechend

- Nuc: einen Rest der Formel OH, OR¹, O-CO-R², SR³ oder NR⁴R⁵, worin
- R¹: Alkyl, Alkenyl, Alkinyl, wobei die letztgenannten drei Reste unsubstituiert oder durch einen oder mehrere Reste aus der Gruppe Halogen, Hydroxy, Alkoxy, Alkylthio, Cyano, Nitro, Carboxy, Carbalkoxy, Amino, Monoalkylamino und Dialkylamino substituiert sind, oder unsubstituiertes oder substituiertes Aryl, unsubstituiertes oder substituiertes Arylalkyl,
vorzugsweise Alkyl, das unsubstituiert oder durch ein oder mehrere Halogenatome oder einen oder mehrere Alkoxyreste substituiert ist, oder unsubstituiertes oder substituiertes Aryl, unsubstituiertes oder substituiertes Arylalkyl,
- R²: Wasserstoff oder einen Rest analog R¹,
vorzugsweise Alkyl, das unsubstituiert oder durch einen oder mehrere Reste aus der Gruppe Halogen, Alkoxy, Alkylthio, Cyano, Nitro, Carbalkoxy und Dialkylamino substituiert ist, oder unsubstituiertes oder substituiertes Aryl, unsubstituiertes oder substituiertes Arylalkyl,
- R³: einen Rest analog R¹,
vorzugsweise Alkyl, das unsubstituiert oder durch einen oder mehrere Reste aus der Gruppe Halogen, Alkoxy und Alkylthio substituiert ist,
- R⁴: Wasserstoff, Acyl oder einen Rest analog R¹,
vorzugsweise Wasserstoff, Alkyl, Acyl oder unsubstituiertes oder substituiertes Aryl, und
- R⁵: Wasserstoff, Acyl oder einen Rest analog R¹,
vorzugsweise Wasserstoff, Alkyl, Acyl oder unsubstituiertes oder substituiertes Aryl,
bedeuten; dabei haben die Bezeichnungen Aryl und Acyl die weiter oben bereits erläuterten Bedeutungen; bei den C-haltigen Substituenten sind solche mit 1 bis 6 C-Atomen, vorzugsweise 1 bis 4 C-Atomen, bevorzugt.

Die Verbindungen der Formel (I) fallen in Form der freien Sulfonsäuren oder in Form ihrer Salze an, je nachdem welcher pH-Wert bei der Isolierung der Verbindungen (I) eingestellt wird. Als Salze eignen sich aus wirtschaftlichen Gründen vor allem die Alkali- und Erdalkalimetallsalze, insbesondere die Alkalimetallsalze, wie Natrium- und Kaliumsalze.

Meistens ist es zweckmäßig, die erfindungsgemäße Umsetzung der inneren Diazoniumsalze (II) in Gegenwart eines organischen Lösungsmittels durchzuführen, das auch ein Gemisch aus mehreren organischen Lösungsmitteln sein kann. Geeignete Lösungsmittel sind z.B. unpolare Lösungsmittel und vorzugsweise polare protische oder aprotisch dipolare Lösungsmittel und deren Mischungen. Wäßrig-organische Lösungsmittel sind im Einzelfall auch erfindungsgemäß einsetzbar, wobei jedoch weitgehend wasserfreie Lösungsmittel bevorzugt verwendet werden, um Nebenreaktionen zu vermeiden.

Beispiele für geeignete Lösungsmittel sind
- Ether, wie Diethylether, Tetrahydrofuran (THF), Dioxan, Diglyme und Tetraglyme,
- Amide, wie Dimethylformamid (DMF), Dimethylacetamid und N-Methylpyrrolidon,
- Ketone, wie Aceton,
- Nitrile, wie Acetonitril, Propionitril, Butyronitril und Benzonitril,
- Sulfoxide und Sulfone, wie Dimethylsulfoxid (DMSO) und Sulfolan,
- halogenierte aliphatische und aromatische Kohlenwasserstoffe, wie Methylenchlorid und Chlorbenzol.
Vor allem in Kombinationen von Lösungsmitteln lassen sich oft auch Alkohole, wie die Alkanole Methanol, Ethanol, n- und i-Propanol, n-, i-, t- und 2-Butanol, einsetzen.

Als Metallkatalysatoren der Gruppe VIII des Periodensystems kommen beispielsweise die Metalle der chemischen Symbole Co, Ni, Pd, Pt, Rh, Ir und Os in Betracht, die in Form der freien Metalle, auf einem Träger (z.B. Silikagel oder Kohle), als Metallsalz (z. B. CoCl₂) oder als Metallkomplex eingesetzt werden können. Diese Katalysatoren sind weitgehend schon als Katalysatoren für Carbonylierungsreaktionen bekannt (siehe obengenannte Literaturstellen).

Beispiele für geeignete Katalysatoren sind Palladiumdiacetat [Pd(OAc)₂], Bis(dibenzylidenaceton)-palladium [ Pd(dba)₂ ], Tetrakis-(triphenylphosphan)palladium [ Pd(P(C₆H₅)₃)₄ ], Tetrakis-(tritolylphosphan)-palladium [ Pd(P(C₇H₇)₃)₄ ], Pd/Kohle, Bis-(triphenylphosphan)-palladium-dichlorid [ Pd(P(C₆H₅)₃)₂Cl₂ ] und Komplexe des Typs Nickel-bis-(diphenylphosphinalkylen)-dichlorid, wobei "alkylen" z. B. C₂-C₄-alkylen bedeutet.

Außerdem kann Kupfer als Katalysator eingesetzt werden, beispielsweise in Form der Cu^{I}- oder Cu^{II}-Salze, wie CuCl₂.

Bevorzugt wird erfindungsgemäß Palladium in den genannten Anwendungsformen als Katalysator eingesetzt, insbesondere Palladiumdiacetat oder Palladium/Kohle.

Das erfindungsgemäße Verfahren wird in der Regel so durchgeführt, daß man zunächst eine Lösung oder Suspension des innere Diazoniumsalzes (II) bereitstellt, beispielsweise durch Auflösen oder Suspendieren des separat hergestellten Salzes oder durch direkte Verwendung der Reaktionsmischung nach Diazotierung der entsprechenden Aminoarensulfonsäure.

Für die Carbonylierung werden, gegebenenfalls nach Zugabe von organischem Lösungsmittel und, falls noch nicht vorhanden, des Nucleophils Nuc-H oder dessen Salz, der nötige Katalysator zugesetzt und Kohlenmonoxid zugeführt. Die Zuführung von CO kann beispielsweise durch Einleiten oder Aufpressen unter Druck erfolgen, wobei die Umsetzungsrate vom jeweiligen Druck abhängt.

Der optimale Druck für die industrielle Verfahrensweise kann durch Vorversuche leicht ermittelt werden und liegt in der Regel von 1 bis 120 bar (10⁵ bis 120• 10⁵ Pascal), vorzugsweise 1 bis 50 bar, insbesondere 3 bis 15 bar. Bevorzugt beziehen sich die Druckangaben auf den Partialdruck des CO. Das Kohlenmonoxid kann beispielsweise als Reingas zugeführt werden; jedoch sind auch Mischgase mit einem Gehalt an CO, z. B. Synthesegas (CO + H₂) oder Gasmischungen von CO mit Stickstoff oder anderen unter den Reaktionsbedingungen inerten Gasen, einsetzbar. Bei Durchführung der Reaktion unter Überdruck sind zweckmäßig übliche Apparaturen wie Druckkessel, Autoklaven, Bombenrohre und ähnliches verwendbar.

Die Reaktionstemperatur für die Carbonylierung sollte so niedrig gewählt werden, daß die Stabilität des Diazoniumsalzes nicht gefährdet ist. Die Carbonylierungsreaktion wird in der Regel im Bereich von -78°C bis zur Zersetzungstemperatur des Diazoniumsalzes durchgeführt, vorzugsweise von -50 bis + 100 °C, insbesondere von -20 bis + 50 °C.

Die Umsetzungsrate kann außerdem vom Mengenverhältnis der Verbindung (II) zur Menge an Katalysator und der Konzentration des Katalysators abhängen; Das Mengenverhältnis läßt sich jedoch meistens in einem großen Bereich variieren. Bezogen auf das Gewicht ist das Verhältnis (II):Katalysator vorzugsweise im Bereich von 10000:1 bis 1:1, insbesondere 1000:1 bis 10:1.

Das Verfahren zur Carbonylierung läßt sich technisch verhältnismäßig einfach batchweise oder kontinuierlich und mit guten Ausbeuten durchführen. Dieses vorteilhafte Resultat war nicht zu erwarten. Überraschenderweise können die inneren Diazoniumsalze (II) erfindungsgemäß eingesetzt werden, ohne daß eine Überführung in Tetrafluorborate erforderlich ist. Außerdem war nicht zu erwarten, daß die aromatischen Sulfonsäuregruppen mit ihrem bekannt starken Einfluß auf die elektronischen Verhältnisse des aromatischen Kerns die Carbonylierungsreaktion als solche und auch die Selektivität der Carbonylierung bei Anwesenheit weiterer prinzipiell reaktionsfähiger Gruppen, wie Halogenatome, praktisch nicht beeinträchtigen würden.

Die erfindungsgemäß in der Carbonylierungsstufe einzusetzenden inneren Diazoniumsalze (II) sind teilweise neu; beispielsweise sind Verbindungen der Formel (II), worin Ar ein durch ein oder zwei lodatome substituiertes Phenylen bedeutet, neu und ebenfalls Gegenstand der Erfindung. Die bekannten und die neuen Verbindungen der Formel (II) können analog bekannten Methoden aus den entsprechenden Aminoarensulfonsäuren hergestellt werden (siehe z.B. Houben-Weyl, Meth. d. Org. Chem. Bd. X/3, S. 16 ff. und Bd. E16/2). Dabei kann man beispielsweise das Diazoniumarensulfonat (II) entweder in wäßrigem Medium mit einem Diazotierreagenz wie salpetriger Säure oder Nitrosylschwefelsäure herstellen, anschließend isolieren und in ein organisches Medium überführen oder man kann das Diazoniumarensulfonat (II) direkt in dem bei der anschließenden Carbonylierungsreaktion zu verwendenden Lösungsmittel mit einem Diazotierungsreagenz wie Alkylnitrit oder Nitrosylschwefelsäure darstellen.

Es ist bekannt, daß sich isolierte Aryldiazoniumsulfonate explosionsartig zersetzen können (Houben-Weyl, Methoden d. org. Chemie, Bd X, 3, S. 32 ff; H. Wichelhaus, Chem. Ber. 1901, 34, 11).

Zur Verbesserung der Stabilität gegen spontane oder explosionsartige Zersetzung können verschiedene Inertstoffe dem inneren Diazoniumsalz, vorzugsweise vor dessen Isolierung oder vor der direkten Weiterverarbeitung der Reaktionsmischung zugesetzt werden.

Als stabilisierende Inertstoffe kommen beispielsweise organische Lösungsmittel in Frage, wie sie bereits oben als Lösungsmittel für die Carbonylierung zur Verbindung (I) erwähnt worden sind, vorzugsweise aromatische Kohlenwasserstoffe wie Xylol oder Nitrile wie Acetonitril oder Alkohole wie t-Butanol. Weiterhin kommen anorganische Inertstoffe, z.B. Mineralsalze oder Kieselgur oder organische Inertstoffe, z.B. Aktivkohle, Polyoxymethylen, Polypropylen oder Polyethylen, in Frage. Die stabilisierenden Inertstoffe werden vorzugsweise der wäßrigen Reaktionsmischung vor der Isolierung der inneren Diazoniumsalze oder vor der direkten Verwendung der Reaktionsmischung für die Carbonylierung zugesetzt.

Die allgemeine Reaktionssequenz für die kombinierte Verfahrensweise, welche die Herstellung des Diazoniumarensulfonats (II) einschließt, ist in Schema 1 am Beispiel von Aminobenzolsulfonsäuren (III) dargestellt:

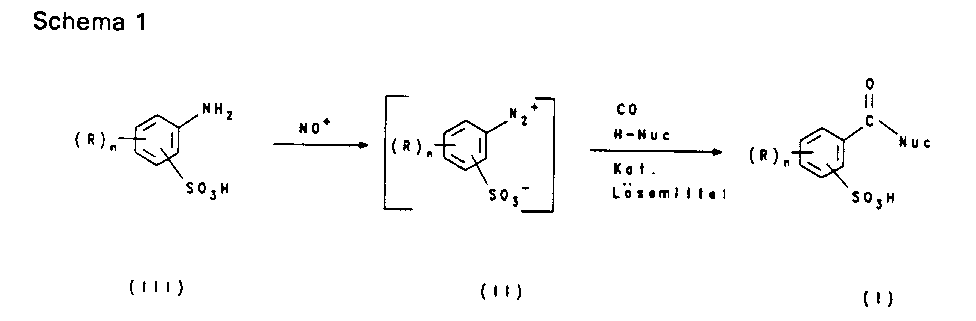

Die zugrundeliegenden Aminoarensulfonsäuren, vorzugsweise Anilinsulfonsäuren (III), sind wohlfeile, im technischen Maßstab herstellbare Verbindungen (siehe beispielsweise Sulfonierung von gegebenenfalls substituierten Anilinen in Houben-Weyl, Meth. d. Org. Chem. Bd. IX, 465ff., 512ff.) oder können aus wohlfeilen einfacher substituierte Aminoarensulfonsäuren bzw. Anilinsulfonsäuren durch eine große Zahl geeigneter Derivatisierungsreaktionen in die gewünschten Verbindungen (III) überführt werden; siehe beispielsweise die lodierung von Anilinsulfonsäuren in J. Chem. Soc. 1909, 1683).

Die nach der Carbonylierung anfallenden Rohprodukte sind Verbindungen der Formel (I) oder deren Salze, je nachdem welcher pH-Wert bei der Isolierung des Produkts eingestellt wird. Zur Aufarbeitung des Reaktionsansatzes können gegebenenfalls die in Labor und Verfahrenstechnik üblichen Methoden zur Isolierung und Reinigung, wie z.B. Kristallisation, Extraktion, Destillation und Chromatographie, angewendet werden. Vor allem bei größeren Ansätzen ist es zweckmäßig, den Katalysator zurückzugewinnen und für den nächsten Ansatz zu verwenden.

In manchen Fällen sind die Produkte der Formel (I) selbst sehr reaktive Zwischenstufen wie z. B. Anhydride oder aktivierte Ester; sie können vorteilhaft entweder direkt oder nach kurzer Zwischenisolierung in einem Folgeschritt zu stabileren Produkten der Formel (I) oder anderen Produkten weiterverarbeitet werden; häufig bietet sich bei Anhydriden als primärem Hauptprodukt zunächst eine alkalische Hydrolyse zur freien Carbonsäure (II), Nuc = OH, an.

Allgemein stellen die Verbindungen der Formel (I) oder deren Salze geeignete Zwischenprodukte zur Herstellung von wichtigen Anwendungsprodukten, wie Arzneimitteln, Pflanzenschutzmitteln und Farbstoffen, dar oder können mit Hilfe einer Vielzahl von Standardmethoden in die gewünschten Zwischenprodukte der obengenannten allgemeinen Formel (A) überführt werden. Von besonderem Interesse sind die Weiterverarbeitung zu Sulfochloriden und Sulfonamiden; siehe beispielsweise Sulfochloride nach Houben-Weyl, Meth. d. Org. Chem. Bd. IX, 564ff. und Sulfonamide nach Houben-Weyl, Meth. d. Org. Chem. Bd. IX, 605ff.

### Beispiele

### 1) 5-lod-carboxy-benzolsulfonsäure-mononatriumsalz

1a) Herstellung des Diazoniumsalzes
   7,5 g (25,1 mmol) 2-Amino-5-iod-benzolsulfonsäure werden in 20 ml Wasser suspendiert und durch Zugabe von 1,64 ml konzentrierter Schwefelsäure und 1,82 g Natriumnitrit in 5 ml Wasser bei 0 °C diazotiert. Anschließend wird 1 h gerührt, abgesaugt, mit Methanol und Diethylether jeweils zweimal gewaschen. Man erhält 2-Diazonium-5-iod-benzolsulfonat als hellgelbes Pulver mit einem Zersetzungspunkt von 143 °C und folgenden NMR-Daten:
   - ¹H-NMR (D₂O): δ [ppm] =: 8,95 (d, 1H); 8,35 (dd, 1H); 8,21 (d, 1H)
1b) Carbonylierung
   Das nach 1a) erhaltene Diazoniumsalz wird in 75 ml Acetonitril suspendiert. Nach Zugabe von 4,92 g Natriumacetat und 0,22 g Palladiumdiacetat wird das Gemisch bei 0 °C in einen Autoklaven gefüllt und nach Aufpressen von 10 bar Kohlenmonoxid für 2 Stunden intensiv geschüttelt. Nach dem Entspannen des Autoklavs wird das Reaktionsgemisch filtriert, der Rückstand mit 50 ml 20 %ige Natronlauge 1 Stunde gerührt und mit konzentrierter Salzsäure auf pH 1 gestellt, wonach das Produkt ausfällt. Nach Trocknen des Produkt werden 5,5 g (63% d. Th.) 5-Iod-carboxy-benzolsulfonsäure-mononatriumsalz als weißes Pulver erhalten. Die Mutterlauge enthält noch größere Mengen an Produkt und kann in den Hydrolyseschritt des nächsten Ansatzes zurückgeführt werden.
   Physikalische Daten des erhaltenen Produkts:
   - Schmelzpunkt (Fp.) von über 280 °C
   - ¹H-NMR (D₂O): δ [ppm] = 8,15 (d, 1H); 7,84 (dd, 1H); 7,19 (d, 1H)

### 2) 5-Iod-2-methoxycarbonyl-benzolsulfonsäure

### Variante 2a)

22,5 g 2-Amino-5-iod-benzolsulfonsäure wurden in 150 ml Acetonitril fein suspendiert und mit 4,18 ml konzentrierter Schwefelsäure versetzt. Man diazotiert mit 7,73 g n-Butylnitrit bei 25 °C und zerstört anschließend den Überschuß an Butylnitrit mit Amidosulfonsäure. Die erhaltene Suspension wird mit 9,13 ml Methanol und 1,0 g Palladium/Kohle (10 %ig, Wassergehalt 50 %ig) versetzt und in einen 1-Liter-Autoklaven gefüllt. Nach Spülen mit Schutzgas wird Kohlenmonoxid mit einem Druck von 10 bar aufgepreßt. Nach einer Reaktionszeit von 2 Stunden unter intensivem Schütteln bei Raumtemperatur wird der Autoklav entspannt, mit Schutzgas gespült und die Reaktionslösung filtriert. Das Filtrat wird unter reduziertem Druck eingedampft, und man erhält 24,6 g (95% d. Th.) 5-lod-2-methoxycarbonyl-benzolsulfonsäure in Form eines dunklen Öls, das beim Stehenlassen langsam kristallisiert. NMR-Daten des Produkts:
- ¹H-NMR (DMSO-d⁶):: δ [ppm] = 8,03 [(d); 1H]; 7,76 [(dd); 1H]; 7,10 [(d); 1H]; 3,72 [(s); 3H]; 9,3 [(s), breit]

### Variante 2b)

16 g eines nach Beispiel 1a) erhaltenen Diazoniumsalzes werden mit 1,0 g Palladium/Kohle (10 %ig, Wassergehalt 50 %ig) und 6,28 ml Methanol versetzt und in einen 1-Liter-Autoklaven gefüllt. Nach Spülen mit Schutzgas wird Synthesegas (50 Vol.-% CO und 50 Vol.-% H₂) mit einem Druck von 20 bar aufgepreßt. Nach einer Reaktionszeit von 4 Stunden unter intensivem Schütteln bei Raumtemperatur wird der Autoklav entspannt, mit Schutzgas gespült und die Reaktionslösung filtriert. Das Filtrat wird unter reduziertem Druck eingedampft, und man erhält 14,2 g (80,4% d. Th.) 5-lod-2-methoxycarbonylbenzolsulfonsäure in Form eines dunklen Öls, das beim Stehenlassen langsam kristallisiert. NMR-Daten des Produkts: wie in Variante 2a).

### Variante 2c)

25 g (81 mmol) 2-Amino-5-iod-benzolsulfonsäure werden in 100 ml Wasser suspendiert und mit 27,8 g (87,6 mmol) Nitrosylschwefelsäure (40 %ig) diazotiert. Nach vollständiger Diazotierung wird der Nitrosyl-Überschuß durch wenig Amidosulfonsäure zerstört.
Zu der erhaltenen Suspension werden 75 g Kieselgur gegeben und nach Zugabe von 50 ml Wasser intensiv untergemischt. Man saugt ab, wäscht den Rückstand je zweimal mit Wasser und Methanol und einmal mit Acetonitril. Die Mischung wird durch Stehenlassen an der Luft getrocknet. Man erhält 97,3 g des inneren Diazoniumsalzes auf Kieselgur, welches in 200 ml Acetonitril suspendiert wird. Man gibt bei 25°C 2 g Palladium/Kohle (Typ Degussa E10 N/W) sowie 8,85 ml (0,21 mol) Methanol zu. Die Mischung wird in einen 1-l-Autoklaven überführt, der mit Kohlenmonoxid zweimal gespült wird. Der Autoklav wird verschlossen und es werden 10 bar CO aufgepreßt. Unter intensivem Rühren läßt man 4 Stunden bei 25°C reagieren. Der Autoklav wird entspannt, zweimal mit Stickstoff gespült und der Inhalt filtriert. Der Rückstand wird mit Acetonitril gewaschen und das gereinigte Filtrat im Vakuum vom Solvens befreit.
Man erhält 25,5 g eines dunklen Öls. Der Reingehalt beträgt 80,1 % an 5-Iod-2-methoxycarbonyl-benzolsulfonsäure.

### Beispiele 3-31

Analog den Beispielen 1 und 2 werden aus entsprechend substituierten Aminobenzolsulfonsäuren (III) die in der folgenden Tabelle 1 aufgeführten Verbindungen der Formel (la) erhalten:

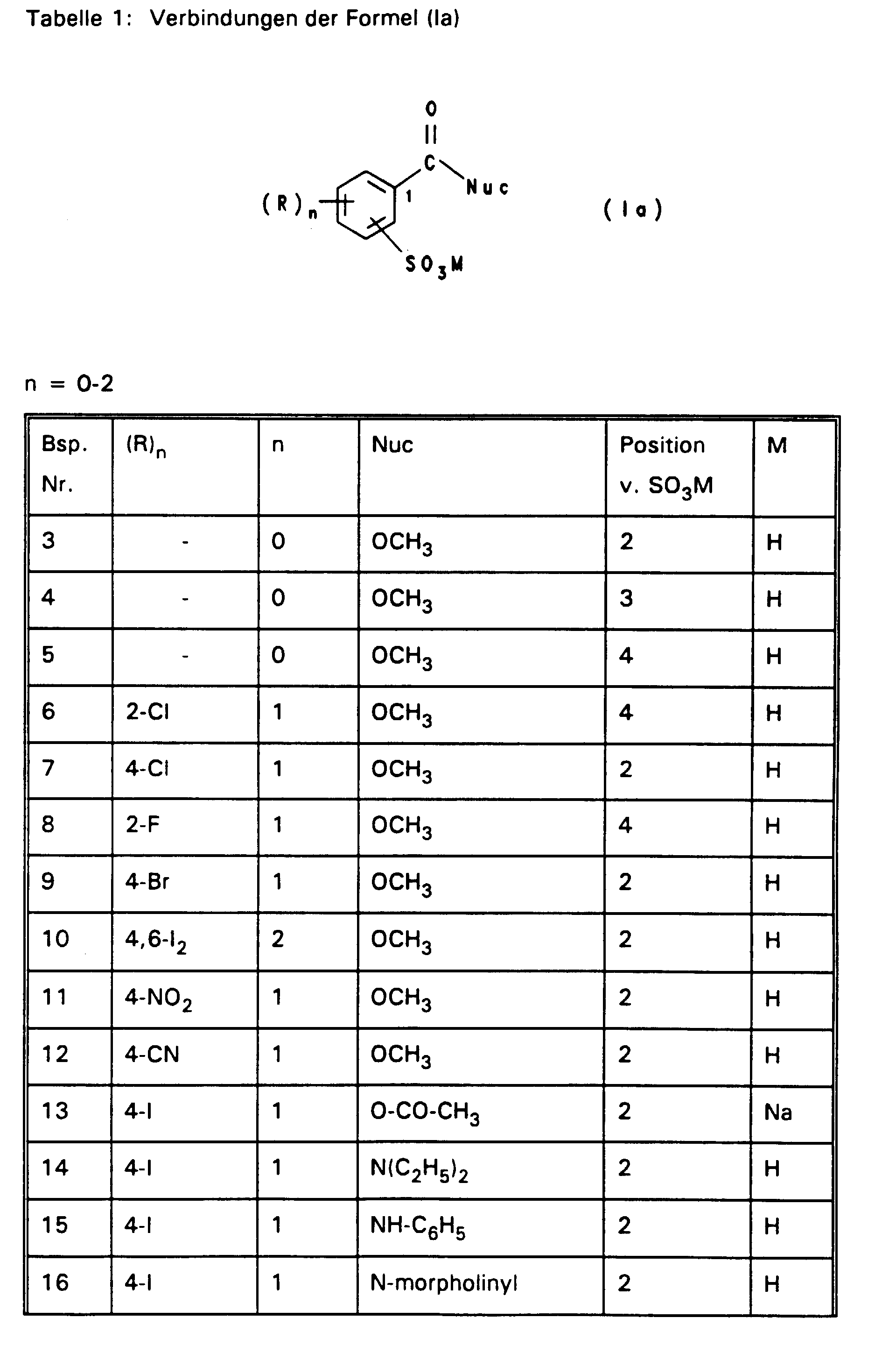

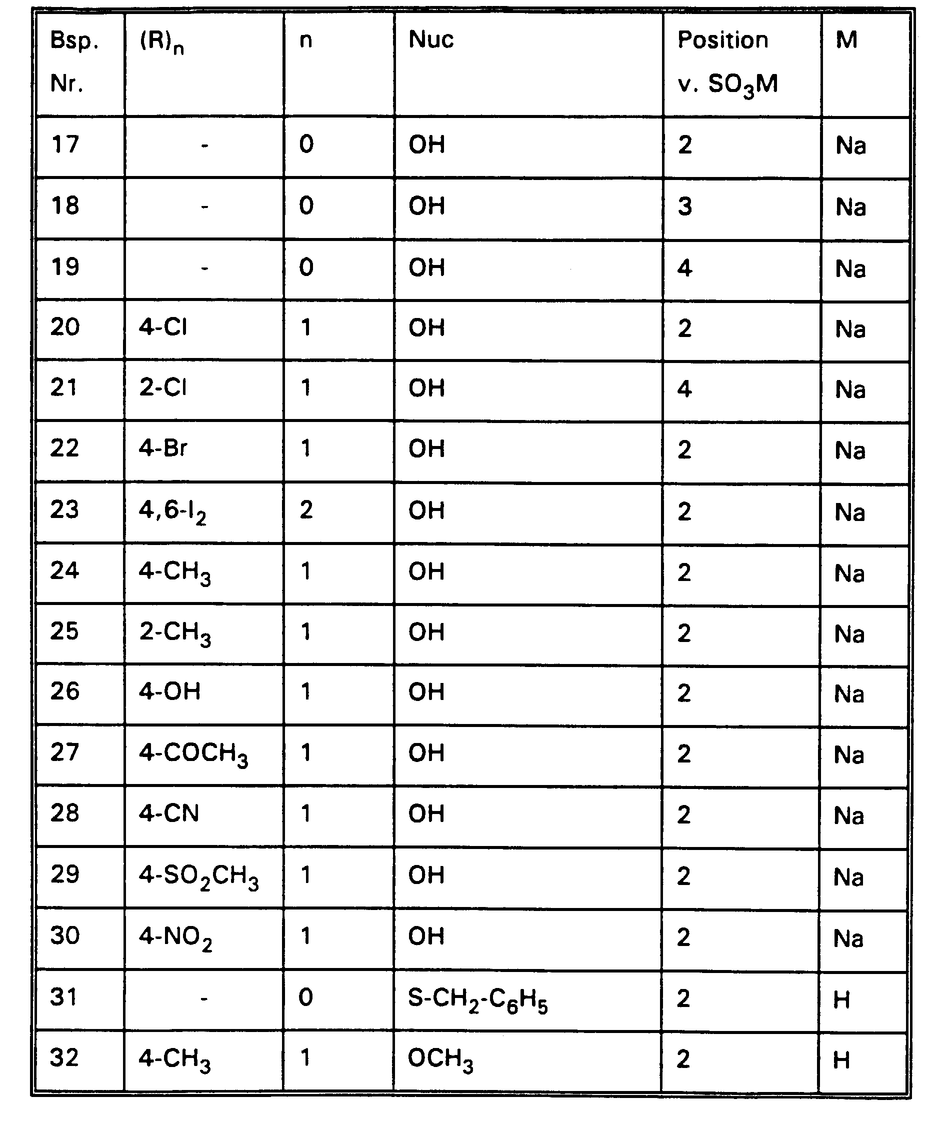

Charakteristische Daten zu Verbindungen aus Tabelle 1:

Die Verbindung aus Beispiel Nr. 3 ist ein Öl mit folgenden chemischen Verschiebungen im ¹H-NMR-Spektrum in DMSO-d⁶:
- δ [ppm] =: 7,74 [dd; 1H]; 7,45 [td; 1H], 7,38 [td; 1H]; 7,28 [dd, 1H]; 3,72 [s; 3H]

Die Verbindung aus Beispiel Nr. 7 ist ein Öl mit folgenden chemischen Verschiebungen im ¹H-NMR-Spektrum in DMSO-d⁶:
- δ [ppm] =: 8,73 [breit; SO₃H]; 7,71 [d; 1H]; 7,49 [dd; 1H]; 7,36 [d; 1H]; 3,73 [s, 3H]

Die Verbindung aus Beispiel Nr. 32 bildet schmierige Kristalle mit folgenden ¹H-NMR-Resonanzen in DMSO-d⁶:
- δ [ppm] =: 7,54 [d; 1H]; 7,51 [breit; SO₃H]; 7,18 [d; 1H]; 7,16 [s; 1H]; 3,69 [s, 3H]; 2,33 [s; 3H]

## Patentansprüche

1. Verfahren zur Herstellung von Verbindungen der allgemeinen Formel (I) oder deren Salzen,
HO₃S-Ar-CO-Nuc (I)
worin
Ar einen unsubstituierten oder substituierten Arylenrest und
Nuc den Rest eines Nukleophils Nuc-H
bedeuten,
dadurch gekennzeichnet, daß man ein inneres Diazoniumsalz der Formel (II)
⁻O₃S-Ar-N₂⁺ (II)
mit Kohlenmonoxid in Gegenwart eines Metallkatalysators aus der Gruppe, die aus Katalysatoren der Metalle der Gruppe VIII des Periodensystems und des Kupfers besteht, und dem Nucleophil der Formel Nuc-H oder dessen Salz umsetzt.

2. Verfahren nach Anspruch 1, dadurch gekennzeichnet, daß Metallkatalysatoren aus der Gruppe Co, Ni, Pd, Pt, Rh, Ir, Os und Cu eingesetzt werden.

3. Verfahren nach Anspruch 1 oder 2, dadurch gekennzeichnet, daß Palladiumkatalysatoren eingesetzt werden.

4. Verfahren nach einem der Ansprüche 1 bis 3, dadurch gekennzeichnet, daß die Umsetzung bei einem Druck von 1 bis 120 bar und einer Temperatur von -78 °C bis zur Zersetzungstemperatur des Diazoniumsalzes (II) durchgeführt wird.

5. Verfahren nach Anspruch 4, dadurch gekennzeichnet, daß der Druck bei 1 bis 50 bar und die Temperatur bei -50 bis + 100 °C ist.

6. Verfahren nach einem der Ansprüche 1 bis 5, dadurch gekennzeichnet, daß die Umsetzung in Gegenwart eines organischen Lösungsmittels durchgeführt wird, das auch ein Gemisch aus mehreren organischen Lösungsmitteln sein kann.

7. Verfahren nach Anspruch 6, dadurch gekennzeichnet, daß das organische Lösungsmittel ein polares protisches oder aprotisch dipolares Lösungsmittel aus der Gruppe, die aus Ethern, Amiden, Ketonen, Nitrilen, Sulfoxiden, Sulfonen, Alkoholen, halogenierten aliphatischen Kohlenwasserstoffen und halogenierten aromatischen Kohlenwasserstoffen besteht, oder deren Mischungen ist.

8. Verfahren nach einem der Ansprüche 1 bis 7, dadurch gekennzeichnet, daß Nuc-H bzw. dessen Salz aus der Gruppe, die aus Alkoholen und deren Salzen, primären und sekundären Aminen, Mercaptanen, Carbonsäuren und deren Salzen, Ammoniak und Wasser besteht, eingesetzt werden.

9. Verfahren nach Anspruch 8, dadurch gekennzeichnet, daß
Nuc OH, OR¹, O-CO-R², SR³ oder NR⁴R⁵,
R¹ Alkyl, Alkenyl, Alkinyl, wobei die letztgenannten drei Reste unsubstituiert oder durch einen oder mehrere Reste aus der Gruppe Halogen, Hydroxy, Alkoxy, Alkylthio, Cyano, Nitro, Carboxy und Carbalkoxy, Amino, Monoalkylamino und Dialkylamino substituiert sind, oder unsubstituiertes oder substituiertes Aryl, unsubstituiertes oder substituiertes Arylalkyl,
R² Wasserstoff oder einen Rest analog R¹,
R³ einen Rest analog R¹,
R⁴ Wasserstoff, Acyl oder einen Rest analog R¹,
R⁵ Wasserstoff, Acyl oder einen Rest analog R¹
bedeuten.

10. Verfahren nach einem der Ansprüche 1 bis 9, dadurch gekennzeichnet, daß Ar einen 1,2-, 1,3- oder 1,4-Phenylenrest, der unsubstituiert oder durch einen oder zwei Reste aus der Gruppe C₁-C₄-Alkyl, C₁-C₄-Haloalkyl, C₂-C₄-Alkenyl, C₂-C₄-Alkinyl, C₁-C₄-Alkoxy, Benzyl, Halogen, Nitro, Cyano, (C₁-C₄-Alkyl)carbonyl, C₁-C₄-Alkylsulfonyl, C₁-C₄-Alkylthio, Phenoxy, Hydroxy, Mono-(C₁-C₄-alkyl)-amino und Di-(C₁-C₄-alkyl)-amino substituiert ist, bedeutet.

11. Verfahren nach einem der Ansprüche 1 bis 10, dadurch gekennzeichnet, daß die Herstellung der erfindungsgemäß in der Carbonylierungsstufe einzusetzenden inneren Diazoniumsalze (II) durch Diazotierung von entsprechenden substituierten Amino-aren-sulfonsäuren erfolgt, wobei entweder
a) die Diazotierung in wäßrigem Medium erfolgt, anschließend die Verbindung (II), gegebenenfalls in Gegenwart eines stabilisierenden Inertstoffs, isoliert und in das in der Carbonylierungsstufe zu benutzende organische Medium überführt wird oder
b) die Diazotierung direkt in dem in der Carbonylierungsstufe zu benutzenden organischen Medium durchgeführt und gegebenenfalls ein stabilisierender Inertstoff zugeführt wird.

12. Verbindungen der Formel (II)
⁻O₃S-Ar-N₂⁺ (II)
worin
Ar ein durch ein oder zwei lodatome substituiertes Phenylen bedeutet.

13. Verfahren zur Herstellung von Verbindungen der Formel (II), wie sie gemäß Anspruch 12 definiert sind, dadurch gekennzeichnet, daß man eine Aminoarensulfonsäure der Formel (III),
HO₃S-Ar-NH₂ (III)
worin Ar wie in Formel (II) definiert ist, oder deren Salze diazotiert.

## Claims

1. A process for the preparation of compounds of the formula (I) or salts thereof
HO₃S-Ar-CO-Nuc (I)
in which
Ar is an unsubstituted or substituted arylene radical, and
Nuc is the radical of a nucleophile Nuc-H,
which comprises reacting an internal diazonium salt of the formula (II)
⁻O₃S-Ar-N₂⁺ (II)
with carbon monoxide in the presence of a metal catalyst from the group composed of catalysts of metals of group VIII of the Periodic System and of copper, and with the nucleophile of the formula Nuc-H or a salt thereof.

2. The process as claimed in claim 1, wherein the metal catalysts employed are selected from the group composed of Co, Ni, Pd, Pt, Rh, Ir, Os and Cu.

3. The process as claimed in claim 1 or 2, wherein palladium catalysts are employed.

4. The process as claimed in any of claims 1 to 3, wherein the reaction is carried out at a pressure of 1 to 120 bar and a temperature of -78°C up to the decomposition temperature of the diazonium salt (II).

5. The process as claimed in claim 4, wherein the pressure is 1 to 50 bar and the temperature is -50 to +100°C.

6. The process as claimed in any of claims 1 to 5, wherein the reaction is carried out in the presence of an organic solvent, which may also be a mixture of a plurality of organic solvents.

7. The process as claimed in claim 6, wherein the organic solvent is a polar protic or aprotic dipolar solvent from the group composed of ethers, amides, ketones, nitriles, sulfoxides, sulfones, alcohols, halogenated aliphatic hydrocarbons and halogenated aromatic hydrocarbons, or mixtures of these.

8. The process as claimed in any of claims 1 to 7, wherein Nuc-H or the salt thereof employed is selected from the group composed of alcohols and their salts, primary and secondary amines, mercaptans, carboxylic acids and their salts, ammonia and water.

9. The process as claimed in claim 8, wherein
Nuc is OH, OR¹, O-CO-R², SR³ or NR⁴R⁵,
R¹ is alkyl, alkenyl or alkynyl, the last-mentioned three radicals being unsubstituted or substituted by one or more radicals selected from the group composed of halogen, hydroxyl, alkoxy, alkylthio, cyano, nitro, carboxyl and carbalkoxy, amino, monoalkylamino and dialkylamino, or is unsubstituted or substituted aryl, or unsubstituted or substituted arylalkyl,
R² is hydrogen or a radical analogous to R¹,
R³ is a radical analogous to R¹,
R⁴ is hydrogen, acyl or a radical analogous to R¹, and
R⁵ is hydrogen, acyl or a radical analogous to R¹.

10. The process as claimed in any of claims 1 to 9, wherein Ar is a 1,2-, 1,3- or 1,4-phenylene radical which is unsubstituted or substituted by one or two radicals selected from the group composed of C₁-C₄-alkyl, C₁-C₄-haloalkyl, C₂-C₄-alkenyl, C₂-C₄-alkynyl, C₁-C₄-alkoxy, benzyl, halogen, nitro, cyano, (C₁-C₄-alkyl)carbonyl, C₁-C₄-alkylsulfonyl, C₁-C₄-alkylthio, phenoxy, hydroxyl, mono-(C₁-C₄-alkyl)amino and di(C₁-C₄-alkyl)amino.

11. The process as claimed in any of claims 1 to 10, wherein the internal diazonium salts (II) to be employed according to the invention in the carbonylation stage are prepared by diazotizing appropriate substituted aminoarenesulfonic acids, where either
a) the diazotization is effected in an aqueous medium and the compound (II) is subsequently isolated, if appropriate in the presence of a stabilizing inert substance, and transferred to the organic medium to be used in the carbonylation stage, or
b) the diazotization is carried out directly in the organic medium to be used in the carbonylation stage and, if appropriate, a stabilizing inert substance is added.

12. A compound of the formula (II)
⁻O₃S-Ar-N₂⁺ (II)
in which
Ar is phenylene which is substituted by one or two iodine atoms.

13. A process for the preparation of compounds of the formula (II) as they are defined in claim 12, which comprises diazotizing an aminoarenesulfonic acid of the formula (III)
HO₃S-Ar-NH₂ (III)
in which Ar is as defined in formula (II), or the salts of this aminoarenesulfonic acid.

## Revendications

1. Procédé de préparation de composés de formule générale (I) ou de sels de ceux-ci,
HO₃S-Ar-CO-Nuc (I)
dans laquelle
Ar représente un groupe aryle non substitué ou substitué, et
Nuc représente le résidu d'un composé nucléophile Nuc-H,
caractérisé en ce que l'on met à réagir un sel interne de diazonium de formule (II)
⁻O₃S-Ar-N₂⁺ (II)
avec du monoxyde de carbone en présence d'un catalyseur métallique choisi parmi le groupe constitué par les catalyseurs des métaux du groupe VIII de la classification périodique des éléments et le cuivre, et avec le nucléophile de formule Nuc-H ou un sel de celui-ci.

2. Procédé selon la revendication 1, caractérisé en ce que l'on utilise des catalyseurs métalliques choisis parmi le groupe Co, Ni, Pd, Pt, Rh, Ir, Os et Cu.

3. Procédé selon la revendication 1 ou 2, caractérisé en ce que l'on utilise des catalyseurs au palladium.

4. Procédé selon l'une des revendications 1 à 3, caractérisé en ce que la réaction est réalisée sous une pression de 1 à 120 bar et à une température de -78°C jusqu'à la température de décomposition du sel de diazonium (II).

5. Procédé selon la revendication 4, caractérisé en ce que la pression est de 1 à 50 bar et la température de -50 jusqu'à +100°C.

6. Procédé selon l'une des revendications 1 à 5, caractérisé en ce que la réaction est réalisée en présence d'un solvant organique, qui peut être également un mélange de plusieurs solvants organiques.

7. Procédé selon la revendication 6, caractérisé en ce que le solvant organique est constitué d'un solvant polaire protique ou dipolaire aprotique choisi parmi les éthers, les amides, les cétones, les nitriles, les sulfoxydes, les sulfones, les alcools, les hydrocarbures aliphatiques halogénés et les hydrocarbures aromatiques halogénés, ou des mélanges de ceux-ci.

8. Procédé selon l'une des revendications 1 à 7, caractérisé en ce que l'on utilise Nuc-H ou son sel choisi parmi le groupe qui est constitué des alcools et de leurs sels, des amines primaires et secondaires, des mercaptans, des acides carboxyliques et de leurs sels, de l'ammoniac et de l'eau.

9. Procédé selon la revendication 8, caractérisé en ce que
Nuc représente un groupe OH, OR¹, O-CO-R², SR³ ou NR⁴R⁵,
R¹ représente un groupe alkyle, alcényle, alcynyle, les trois derniers groupes cités étant non substitués ou substitués par un ou plusieurs résidus choisis parmi un atome d'halogène, un groupe hydroxy, alkoxy, alkylthio, cyano, nitro, carboxy et carbaloxy, amino, monoalkylamino et dialkylamino, ou un groupe aryle non substitué ou substitué, un groupe arylalkyle non substitué ou substitué,
R² représente un atome d'hydrogène ou un groupe analogue à R¹,
R³ représente un groupe analogue à R¹,
R⁴ représente un atome d'hydrogène, un groupe acyle ou un groupe analogue à R¹,
R⁵ représente un atome d'hydrogène, un groupe acyle ou un groupe analogue à R¹.

10. Procédé selon l'une des revendications 1 à 9, caractérisé en ce que Ar représente un groupe 1,2-, 1,3-ou 1,4-phénylène, qui est non substitué ou substitué par un ou deux groupes choisis parmi le radical alkyle en C₁-C₄, halogénoalkyle en C₁-C₄, alcényle en C₂-C₄, alcynyle en C₂-C₄, alkoxy en C₁-C₄, benzyle, un atome d'halogène, un groupe nitro, cyano, (alkyle en C₁-C₄)-carbonyle, alkylsulfonyle en C₁-C₄, alkylthio en C₁-C₄, phénoxy, hydroxy, mono-(alkyle en C₁-C₄)-amino et di-(alkyle en C₁-C₄)-amino.

11. Procédé selon l'une des revendications 1 à 10, caractérisé en ce que l'on effectue la préparation des sels internes de diazonium (II) à utiliser selon l'invention dans l'étape de carbonylation,par diazotation d'acides aminoarène-sulfoniques substitués correspondants, dans lequel
a) soit on réalise la diazotation en milieu aqueux, on sépare ensuite le composé (II), éventuellement en présence d'une matière inerte stabilisante, et dans lequel on l'introduit dans le milieu organique à utiliser dans l'étape de carbonylation, soit
b) on effectue la diazotation directement dans le milieu organique à utiliser pour l'étape de carbonylation et on ajoute éventuellement une matière inerte stabilisante.

12. Composés de formule (II)
⁻O₃S-Ar-N₂⁺ (II)
dans laquelle
Ar représente un groupe phénylène substitués par un ou deux atomes d'iode.

13. Procédé de préparation de composés de formule (Il), tels qu'ils sont définis selon la revendication 12, caractérisé en ce que l'on réalise la diazotation d'un acide aminoarène-sulfonique de formule (III),
HO₃S-Ar-N₂⁺ (III)
dans laquelle Ar est défini comme dans la formule (II), ou de sels de celui-ci.
